# EUROPEAN PATENT APPLICATION

(11) **EP 1 788 502 A1**
(43) Date of publication of application: **23.05.2007**
(21) Application number: 06124281.4
(22) Date of filing: 17.11.2006
(51) Int. Cl.: G06F 19/00

(54) **Computer supported collaborative work system providing simultaneous manipulation of shared data in a healthcare environment**

(30) Priority: 22.11.2005 US 739526 P; 17.01.2006 US 333124
(71) Applicant: General Electric Company, Schenectady, NY 12345 (US)
(72) Inventor: Mahesh, Prakash, Hoffman Estates, IL 60192 (US); Gentles, Thomas A., Algonquin, IL 60102 (US); Morita, Mark M., Arlington Heights, IL 60005 (US); Ricard, Mark, Naperville, IL 60564 (US); Karlathungal, Murali Kumaran, Hoffman Estates, IL 60194 (US)
(74) Representative: Bedford, Grant Richard

(57) **Abstract**

The present invention provides a system (400) for improved conferencing and collaboration in a healthcare environment. The system (400) includes an initiator workstation (410) capable of sharing data over a network with a participant workstation (420) and manipulating the shared data based at least in part on input from an initiator. The system also includes a participant workstation (420) capable of manipulating the shared data based at least in part on input from a participant. The initiator and participant workstations (410,420) are capable of simultaneous user manipulation of the shared data.

## Description

The present invention generally relates to an image and information management system. In particular, the present invention relates to an image and information management system with improved conferencing and collaboration capability.

A clinical or healthcare environment is a crowded, demanding environment that would benefit from organization and improved ease of use of imaging systems, data storage systems, and other equipment used in the healthcare environment. A healthcare environment, such as a hospital or clinic, encompasses a large array of professionals, patients, and equipment. Personnel in a healthcare facility must manage a plurality of patients, systems, and tasks to provide quality service to patients. Healthcare personnel may encounter many difficulties or obstacles in their workflow.

In a healthcare or clinical environment, such as a hospital, a large number of employees and patients may result in confusion or delay when trying to reach other medical personnel for examination, treatment, consultation, or referral, for example. A delay in contacting other medical personnel may result in further injury or death to a patient. Additionally, a variety of distractions in a clinical environment may frequently interrupt medical personnel or interfere with their job performance. Furthermore, workspaces, such as a radiology workspace, may become cluttered with a variety of monitors, data input devices, data storage devices, and communication devices, for example. Cluttered workspaces may result in inefficient workflow and service to clients, which may impact a patient's health and safety or result in liability for a healthcare facility.

Data entry and access is also complicated in a typical healthcare facility. Speech transcription or dictation is typically accomplished by typing on a keyboard, dialing a transcription service, using a microphone, using a Dictaphone, or using digital speech recognition software at a personal computer. Such dictation methods involve a healthcare practitioner sitting in front of a computer or using a telephone, which may be impractical during operational situations. Similarly, for access to electronic mail or voice messages, a practitioner must typically use a computer or telephone in the facility. Access outside of the facility or away from a computer or telephone is limited.

Thus, management of multiple and disparate devices, positioned within an already crowded environment, that are used to perform daily tasks is difficult for medical or healthcare personnel. Additionally, a lack of interoperability between the devices increases delay and inconvenience associated with the use of multiple devices in a healthcare workflow. The use of multiple devices may also involve managing multiple logons within the same environment. A system and method for improving ease of use and interoperability between multiple devices in a healthcare environment would be highly desirable.

Healthcare environments, such as hospitals or clinics, include clinical information systems, such as hospital information systems (HIS), radiology information systems (RIS), clinical information systems (CIS), and cardiovascular information systems (CVIS), and storage systems, such as picture archiving and communication systems (PACS), library information systems (LIS), and electronic medical records (EMR). Information stored may include patient medical histories, imaging data, test results, diagnosis information, management information, and/or scheduling information, for example. The information may be centrally stored or divided among a plurality of locations. Healthcare practitioners may desire to access patient information or other information at various points in a healthcare workflow. For example, during surgery, medical personnel may access patient information, such as images of a patient's anatomy, that are stored in a medical information system. Alternatively, medical personnel may enter new information, such as history, diagnostic, or treatment information, into a medical information system during an ongoing medical procedure.

Imaging systems are complicated to configure and to operate. Often, healthcare personnel may be trying to obtain an image of a patient, reference or update patient records or diagnosis, and/or ordering additional tests or consultation, for example.

Thus, there is a need for a system and method that facilitate operation and interoperability of an imaging system and related devices by an operator.

Additionally, in a healthcare workflow, healthcare providers often consult or otherwise interact with each other. Such interaction typically involves paging or telephoning another practitioner. Thus, interaction between healthcare practitioners may be time- and energy-consuming. Therefore, there is a need for a system and method to simplify and improve communication and interaction between healthcare practitioners.

Furthermore, healthcare practitioners may want or need to review diagnoses and/or reports from another healthcare practitioner. For example, a referring physician may want to review a radiologist's diagnosis and report with the radiologist and/or a technician. As another example, an emergency room physician may need to review results of an emergency room study with the radiologist and/or a family physician. Thus, there is a need for a system and method for notifying or informing appropriate parties of results in order to collaborate for diagnosis and/or treatment review for safe and effective treatment.

Typically, healthcare practitioners determine each other's availability and schedule a collaboration event. Thus, current systems and methods require more manual involvement and multiple steps. Current systems encouraging interactions between healthcare practitioners consist of several discrete or manual actions involving a number of disparate systems and/or individuals. First, third parties are notified of information availability. Then, third parties obtain the information by accessing one or more systems. After a system verifies that the information has been received, the practitioner and third party must determine their availability for collaboration. After the parties schedule a mutually available time for collaboration, the parties may finally collaborate to review exam results, diagnosis, treatment, etc. The involvement of a plurality of disparate systems/parties and requirement of several disparate steps renders current systems and methods complicated, inefficient, and time consuming. An ability to reduce the number of actions required by interested parties, reduce the number of ineffective actions, and reduce the waiting time required to obtain necessary information and perform a collaboration would result in more efficient and effective healthcare delivery.

Healthcare experts are located around the world and are often separated by large distances. Collaboration between experts and other healthcare practitioners is often difficult to coordinate. Additionally, current collaboration systems and efforts do not allow efficient sharing of information, including diagnostic images, between healthcare practitioners. Current communication systems only allow basic textual communication, rather than detail interaction and collaboration between parties. Current systems are limited in their ability to display diagnostic quality images.

Current systems for collaboration and conferencing, such as Microsoft Net Meeting^{™}, typically include phone and/or personal conversations, screen sharing, and/or instant messaging. With respect to phone and/or personal conversations, different users have to login and pull up the context manually. Additionally, explanations have to be done verbally. With respect to screen sharing, only one person has control of the interaction. With respect to instant messaging, communication is poor because it is limited to text. Current collaboration and conferencing systems are not conducive to a healthcare environment because such systems lack the necessary safety and security of such an environment.

Thus, there is a need for a system and method for improved collaboration and conferencing in a healthcare environment.

Current communication/collaboration applications are limited to displaying information and data that is currently displayed on a shared desktop. Relevant information is not always accessible on the shared desktop, so navigation and drill down would be required to extract the information. Relevant information is typically sent asynchronously as in textual reports, images, and other relevant information. The sender of this information is typically not able to communicate live with the recipient regarding questions or concerns on the data. Multimedia reports are typically sent asynchronously, but have no immediate mechanism for synchronous follow-up. Consequently, current communication/collaboration systems are not only inefficient, but may contribute to unnecessary medical errors (improper diagnosis and/or treatment).

Tang et al. (U.S. Pat. No. 5,960,173) discloses a system and method enabling awareness of others working on similar tasks in a computer work environment. Tang et al. discloses awareness of other users. Tang et al. does not disclose real-time sharing of information. Lu et al. (U.S. Pat. App. Pub. No. 2002/0054044) discloses a collaborative screen sharing system. Lu et al. does not disclose contextual, rules-based aggregation of information. Shea et al. (U.S. Pat. App. Pub. No. 2003/0208459) discloses a collaborative context information management system. Shea et al. does not disclose real-time collaboration and contextual information sharing.

Thus, there is a need for a real-time, synchronous communication system by providing immediate context and consult capability to healthcare providers.

Certain embodiments of the present invention provide a system for improved conferencing and collaboration in a healthcare environment. The system includes an initiator workstation capable of sharing data over a network with a participant workstation and manipulating the shared data based at least in part on input from an initiator. The system also includes a participant workstation capable of manipulating the shared data based at least in part on input from a participant. The initiator and participant workstations are capable of simultaneous user manipulation of the shared data.

In an embodiment, the initiator and participant workstations may include picture archiving and communications system workstations. In an embodiment, the initiator and participant workstations may include personal computers. In an embodiment, the shared data may include medical studies, reports, diagnostic images, image annotations, regions of interest, audio, and/or video. In an embodiment, the shared data may be manipulated by an input device or by voice command. In an embodiment, the network may include client-server networks, peer-to-peer networks, wireless networks, and/or the Internet. In an embodiment, the initiator and participant workstations are capable of displaying the shared data.

Certain embodiments of the present invention provide a method for improved conferencing and collaboration in a healthcare environment. The method includes initiating a collaboration session. The collaboration session includes sharing data over a network between an initiator workstation and a participant workstation. The method also includes allowing manipulation of the shared data simultaneously in the collaboration session based at least in part on input from an initiator and a participant.

In an embodiment, the method may further include displaying and/or saving the simultaneous user manipulations. In an embodiment, the method may further include tracking user and/or data information.

Certain embodiments of the present invention provide a computer-readable storage medium. The computer-readable storage medium includes a set of instructions for a computer. The set of instructions includes an initiation routine configured to initiate a collaboration session. The collaboration session includes sharing data over a network between an initiator workstation and a participant workstation. The set of instructions also includes a manipulation routine configured to allow manipulation of the shared data by both the initiator workstation and the participant workstation in real time based at least in part on input from an initiator and a participant.

In an embodiment, the data may be selected based at least in part on one or more rules and/or preferences. The one or more rules and/or preferences may be based at least in part on context information. The context information may include symptoms, diagnoses, treatments, and/or users. In an embodiment, the set of instructions may further include a conflict resolution routine configured to resolve a conflict between real time user manipulations of the shared data. The conflict may be resolved based at least in part on input from the initiator and/or participant. The conflict may be resolved based at least in part on one or more rules and/or preferences.

Various aspects and embodiments of the present invention will now be described in connection with the accompanying drawings, in which:
Figure 1 illustrates an exemplary Picture Archiving and Communication System (PACS) system in accordance with an embodiment of the present invention.
Figure 2 illustrates an image management and communication system with remote control capability in accordance with an embodiment of the present invention.
Figure 3 illustrates a flow diagram of a method for remote control between workstations in accordance with an embodiment of the present invention.
Figure 4 illustrates an image management and communication system with simultaneous collaboration capability in accordance with an embodiment of the present invention.
Figure 5 illustrates a flow diagram for a method for simultaneous collaboration between workstations in accordance with an embodiment of the present invention.
Figure 6 illustrates a graphical user interface for an image and information management system in accordance with an embodiment of the present invention.
The foregoing summary, as well as the following detailed description of certain embodiments of the present invention, will be better understood when read in conjunction with the appended drawings. For the purpose of illustrating the invention, certain embodiments are shown in the drawings. It should be understood, however, that the present invention is not limited to the arrangements and instrumentality shown in the attached drawings.

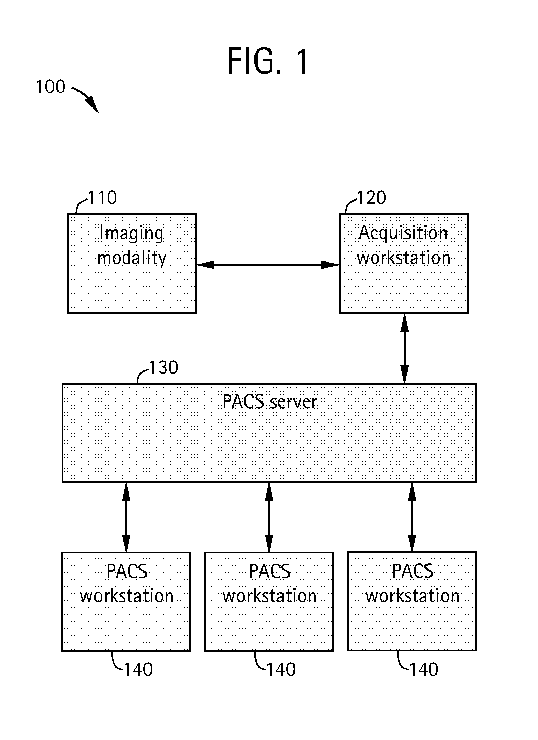

Figure 1 illustrates an exemplary Picture Archiving and Communication System (PACS) system 100 in accordance with an embodiment of the present invention. The PACS system 100 includes an imaging modality 110, an acquisition workstation 120, a network server 130, and one or more display workstations 140. The system 100 may include any number of imaging modalities 110, acquisition workstations 120, network servers 130 and display workstations 140 and is not in any way limited to the embodiment of system 100 illustrated in Figure 1.

In operation, the imaging modality 110 obtains one or more images of a patient anatomy. The imaging modality 110 may include any device capable of capturing an image of a patient anatomy such as a medical diagnostic imaging device. For example, the imaging modality 110 may include an X-ray imager, ultrasound scanner, magnetic resonance imager, or the like. Image data representative of the image(s) is communicated between the imaging modality 110 and the acquisition workstation 120. The image data may be communicated electronically over a wired or wireless connection, for example.

In an embodiment, the acquisition workstation 120 may apply one or more preprocessing functions to the image data in order to prepare the image for viewing on a display workstation 140. For example, the acquisition workstation 120 may convert raw image data into a DICOM standard format or attach a DICOM header. Preprocessing functions may be characterized as modality-specific enhancements, for example (e.g., contrast or frequency compensation functions specific to a particular X-ray imaging device), applied at the beginning of an imaging and display workflow. The preprocessing functions may differ from processing functions applied to image data in that the processing functions are not modality specific and are instead applied at the end of the imaging and display workflow (for example, at a display workstation 140).

The image data may then be communicated between the acquisition workstation 120 and the network server 130. The image data may be communicated electronically over a wired or wireless connection, for example.

The network server 130 may include computer-readable storage media suitable for storing the image data for later retrieval and viewing at a display workstation 140. The network server 130 may also include one or more software applications for additional processing and/or preprocessing of the image data by one or more display workstations 140, for example.

One or more display workstations 140 are capable of or configured to communicate with the server 130. The display workstations 140 may include a general purpose processing circuit, a network server 130 interface, a software memory, and/or an image display monitor, for example. The network server 130 interface may be implemented as a network card connecting to a TCP/IP based network, but may also be implemented as a parallel port interface, for example.

The display workstations 140 may retrieve or receive image data from the server 130 for display to one or more users. For example, a display workstation 140 may retrieve or receive image data representative of a computed radiography (CR) image of a patient's chest. A radiologist may then examine the image for any objects of interest such as tumors, lesions, etc.

The display workstations 140 may also be capable of or configured to apply processing functions to image data. For example, a user may desire to apply processing functions to enhance features within an image representative of the image data. Processing functions may therefore adjust an image of a patient anatomy in order to ease a user's diagnosis of the image. Such processing functions may include any software-based application that may alter a visual appearance or representation of image data. For example, a processing function can include any one or more of flipping an image, zooming in an image, panning across an image, altering a window and/or level in a grayscale representation of the image data, and altering a contrast and/or brightness an image.

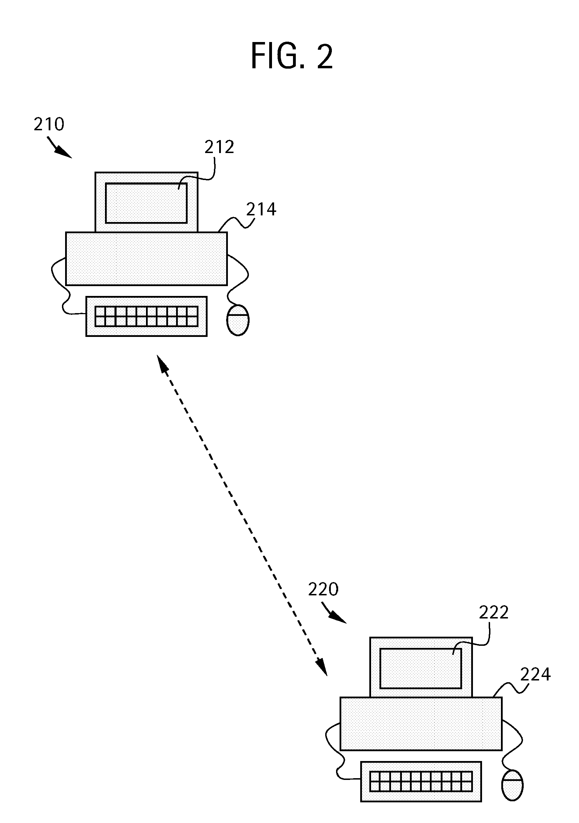

Figure 2 illustrates an image and information management system 200 with remote control capability in accordance with an embodiment of the present invention. The image and information management system 200 includes a plurality of workstations 210, 220. In an embodiment, the image and information management system 200 is a picture archiving and communication system (PACS) including a plurality of PACS workstations. The image and information management system 200 may be a PACS system similar to the PACS system 100 described above in relation to Figure 1.

The image and information management system 200 is capable of performing image management, image archiving, exam reading, exam workflow, and/or other medical enterprise workflow tasks, for example. In an embodiment, the system 200 is or includes a PACS, for example. The system 200 may also include a healthcare or hospital information system (HIS), a radiology information system (RIS), a clinical information system (CIS), a cardiovascular information system (CVIS), a library information system (LIS), order processing system, and/or an electronic medical record (EMR) system, for example. The image management system 200 may include additional components such as an image manager for image management and workflow and/or an image archive for image storage and retrieval.

The image and information management system 200 may interact with one or more modalities, such as an x-ray system, computed tomography (CT) system, magnetic resonance (MR) system, ultrasound system, digital radiography (DR) system, positron emission tomography (PET) system, single photon emission computed tomography (SPECT) system, nuclear imaging system, and/or other modality. The image and information management system 200 may acquire image data and related data from the modality for processing and/or storage.

In an embodiment, one of the workstations 210 may function as an initiator workstation and another of the workstations 220 may function as a slave workstation. The initiator workstation 210 initiates a request to take control of the slave workstation 220. The slave workstation 220 accepts a request for control and allows the initiator workstation 210 to control some or all functionality of the slave workstation 220. In an embodiment, any workstation in the system 200 may serve as an initiator and/or a slave with respect to another workstation.

The initiator workstation 210 may be used to display content and/or activity from the initiator workstation 210 at the slave workstation 220, for example. For example, studies, reports, images, annotations, regions of interest, audio, video, text, and/or other information may be displayed at the slave workstation 220 at the instruction of the initiator workstation 210. Thus, a healthcare practitioner, such as a radiologist, may view content at the slave workstation 220 displayed by the initiator workstation 210. Information from the initiator workstation 210 may be displayed in near real-time at the slave workstation 220. Conferencing features of the system 200 help improve resident workflow, expert consultation, and/or teaching hospitals, for example.

In an embodiment, connection and collaboration between the initiator workstation 210 and the slave workstation 220 occur regardless of display resolution (low resolution display, high resolution display, etc.) at the workstations 210, 220. For example, diagnostic images may be displayed at the initiator workstation 210 and/or slave workstation 220 without regard to display resolution. Software and/or hardware running on the initiator workstation 210 and/or the slave workstation 220 accommodate for differences in display resolution and help to ensure that a diagnostic quality image is displayed. Furthermore, in an embodiment, connection and collaboration between the initiator workstation 210 and the slave workstation 220 occur independent of a number of displays connected to each workstation 210, 220. For example, the system 200 may resolve display of information between an initiator workstation 210 with one or more displays and a slave workstation 220 with one or more displays.

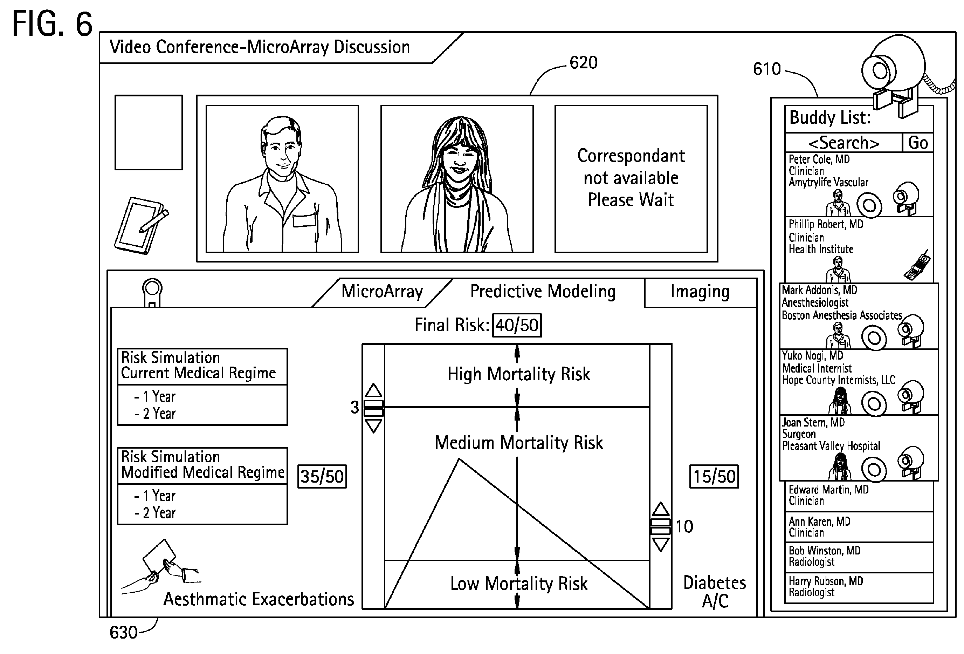

In an embodiment, the initiator workstation 210 includes an interface 212 capable of allowing control of and exchange of information with the slave workstation 220. The interface 212 may be a graphical user interface (GUI), such as the graphical user interface 600 of Figure 6, or other user interface that may be configured to allow a user to access functionality at the initiator workstation 210 and/or the slave workstation 220. The slave workstation 220 may also include an interface 222 that may be configured to allow a user to access functionality at the slave workstation 220. The interfaces 212, 222 may be connected to an input device, such as a keyboard, mousing device, and/or other input device, for example.

Additionally, the initiator workstation 210 and the slave workstation 220 may include communication devices 214 and 224, respectively, to allow communication between the initiator workstation 210 and the slave workstation 220. The communication devices 214, 224 may include a modem, wireless modem, cable modem, Bluetooth^{™} wireless device, infrared communication device, wired communication device, and/or other communication device, for example. The communication devices 214, 224 communicate and transfer data via one or more communication protocols, such as the DICOM protocol. The communication devices 214, 224 coordinate with processors in the workstations 210, 220 to establish a connection between the workstations 210, 220 and remotely execute functionality and/or transfer data, for example.

In an embodiment, the initiator workstation 210 may interface with and/or control the slave workstation 220 according to one or more rules and/or preferences. A password and/or other authentication, such as voice or other biometric authentication, may be used to establish a connection between the initiator workstation 210 and the slave workstation 220.

In an embodiment, users at the workstations 210, 220 may communicate via telephone, electronic "chat" or messaging, Voice over Internet Protocol (VoIP) communication, or other communication via the workstations 210, 220 and/or separate from the workstations 210, 220. Users at the initiator 210 and slave 220 workstations may share display protocols, perspectives, rules, information, etc.

In an embodiment, one or more initiator workstations 210 may communicate with one or more slave workstations 220. The initiator workstation 210 or other component of the system 200 may store profile(s) and/or other connection information for one or more slave workstations 220 or users. In an embodiment, interaction between the initiator workstation 210 and the slave workstation 220 is manually initiated. In an embodiment, interaction between the initiator workstation 210 and the slave workstation 220 may be scheduled based on calendar or availability information, user preference, rules, and/or other criteria, for example. In an embodiment, the slave workstation 220 is automatically detected by the initiator workstation 210. In an embodiment, a certain type of initiator workstation 210, such as a PACS workstation, may communicate with and control a different type of slave workstation 220, such as a HIS, RIS, CIS, CVIS, LIS, or EMR workstation.

In an embodiment, actions that may be controlled by the initiator 210 may be defined as super initiator actions and specialized initiator actions. Super initiation allows control of all functionality at the slave workstation, such as image display, default display protocol (DDP) configuration, report creation/modification, dictation, etc. Specialized initiation allows control of selected functions specified by the slave workstation 220. In an embodiment, functions may be selected at the slave workstation 220 during a response by the slave workstation 220 to a control request from the initiator workstation 210. The slave workstation 220 may specify whether control may be taken as super initiator control or specialized initiator control, for example. If control is specialized user control, the slave workstation 220 selects functions and/or sets of functions that the initiator 210 is allowed to control.

For example, the initiator workstation 210 may be selectively authorized by the slave workstation 220 to display images and adjust display configuration parameters. The initiator workstation 210 may be selectively authorized to control reporting functionality at the slave workstation 220, for example. Alternatively, the initiator workstation 210 may have complete control of the functionality of the slave workstation 210 including image acquisition, image display, image processing, reporting, etc.

In an embodiment, a healthcare practitioner may use the initiator workstation 210 to perform a variety of functions at the slave workstation 220 for another healthcare practitioner. For example, a radiologist may indicate findings within image data at the slave workstation 220 via the initiator workstation 210 for a physician. A healthcare practitioner may also convey and/or identify diagnosis information, treatment information, and/or consultation or referral information, for example. For example, a surgeon may consult a specialist in real-time during surgery and allow the specialist to view and comment on images and/or data from the operation in progress. In an embodiment, a healthcare practitioner may dictate and/or annotate an image or report on the slave workstation 220 via the initiator workstation 210. In an embodiment, functions at the slave workstation 220 may be controlled via voice command at the initiator workstation 210.

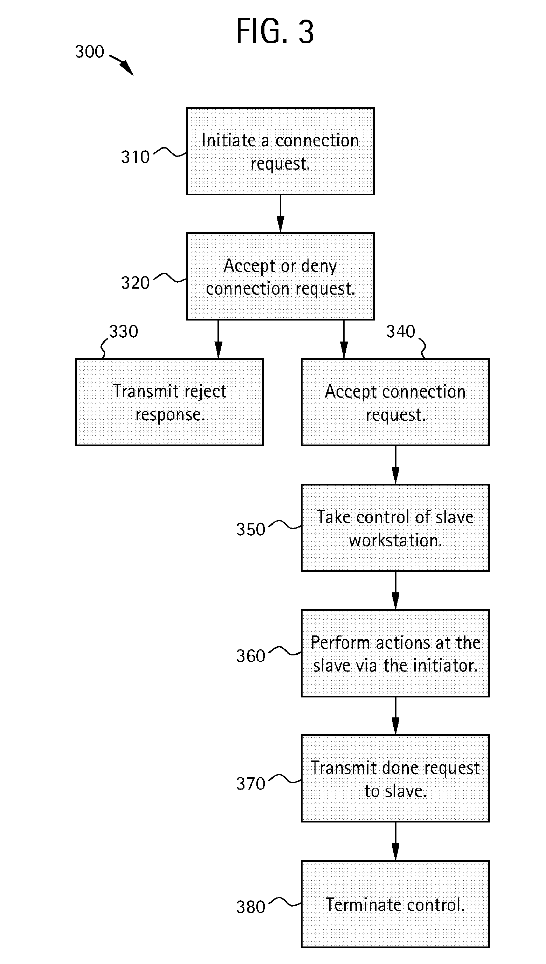

Figure 3 illustrates a flow diagram of a method 300 for remote control between workstations in accordance with an embodiment of the present invention. First, at step 310, a healthcare practitioner initiates a request for connection to a slave workstation. For example, a radiologist initiates a request to perform Centricity PACS workstation conferencing on a second workstation. Next, at step 320, a healthcare practitioner at the slave workstation determines whether to accept or deny the connection request. For example, a radiologist at the second workstation decides whether to accept or deny the request from the Centricity PACS workstation.

Then, at step 330, if the connection request is denied, the slave workstation transmits a reject response, and the request is aborted. In an embodiment, a second slave workstation may then be queried, and/or the connection request may be rescheduled for a later attempt. At step 340, the connection request is accepted.

Then, at step 350, the initiator takes control of the slave workstation. In an embodiment, the initiator workstation controls all or a subset of functionality and data at the slave workstation. An extent of control by the initiator may be defined by user selection, rules, preferences, and/or other parameters, for example. Next, at step 360, allowed actions are performed on the slave workstation via the initiator workstation. For example, the radiologist using the initiator workstation displays and annotates examination results on the slave workstation.

At step 370, a done request is transmitted to the slave workstation. For example, after a conference has concluded, the initiator workstation transmits a done request or end of conference message to the slave workstation. Then, at step 380, control is terminated. For example, the connection established between the initiator workstation and the slave workstation may be ended. In an embodiment, control of the slave workstation is relinquished by the initiator workstation while the connection between the slave workstation and the initiator workstation is maintained.

Thus, certain embodiments provide healthcare practitioners, such as radiologists and residents, with an ability to conference and collaborate remotely. Certain embodiments improve resident workflow by allowing residents to consult in real-time or substantially real-time with senior physicians or specialists. Certain embodiments allow healthcare practitioners to consult with experts in a given field and receive a rapid response from experts around the world. In teaching hospitals or other training or learning environments, education and training may be facilitated by sharing patient data and images with faculty, students, and other healthcare practitioners in a non-classroom environment. Certain embodiments allow peers to share patient information and images for real-time or substantially real-time reading and analysis. Additionally, certain embodiments allow practitioners to conference and share diagnostic quality images.

Certain embodiments allow a user at a workstation, such as a PACS workstation, to take control of another system to display images, create/modify reports, configure a display protocol, and/or execute other functions or share other data at another workstation. Certain embodiments allow collaboration and conferencing between workstations independent of a number of monitors on a workstation. Certain embodiments allow collaboration and conferencing independent of monitor resolutions and/or display protocols. Certain embodiments allow sharing of diagnostic quality images. Additionally, certain embodiments allow real-time or substantially real-time sharing of peer workstation activities.

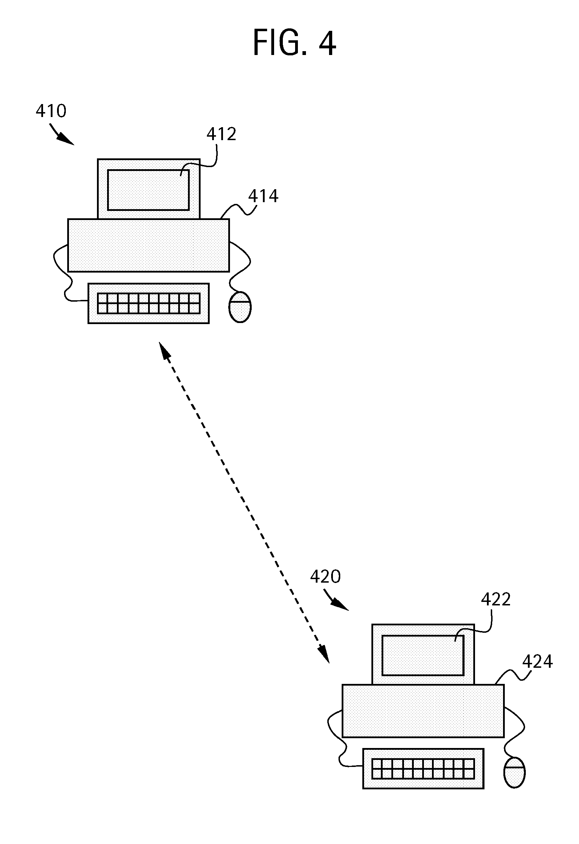

Figure 4 illustrates an image and information management system 400 with simultaneous collaboration capability in accordance with an embodiment of the present invention. The image and information management system 400 includes a plurality of workstations 410, 420. In an embodiment, the image and information management system 400 is a picture archiving and communication system (PACS) including a plurality of PACS workstations. The image and information management system 400 may be a PACS system similar to the PACS system 100 described above in relation to Figure 1.

The image and information management system 400 is capable of performing image management, image archiving, exam reading, exam workflow, and/or other medical enterprise workflow tasks, for example. In an embodiment, the image and information management system 400 is or includes a PACS, for example. The image and information management system 400 may also include a healthcare or hospital information system (HIS), a radiology information system (RIS), a clinical information system (CIS), a cardiovascular information system (CVIS), a library information system (LIS), order processing system, and/or an electronic medical record (EMR) system, for example. The image and information management system 200 may include additional components, such as an image manager for image management and workflow and/or an image archive for image storage and retrieval.

The image and information management system 400 may interact with one or more modalities, such as an x-ray system, computed tomography (CT) system, magnetic resonance (MR) system, ultrasound system, digital radiography (DR) system, positron emission tomography (PET) system, single photon emission computed tomography (SPECT) system, nuclear imaging system, and/or other modality. The image and information management system 400 may acquire image data and related data from the modality for processing and/or storage.

In an embodiment, one of the workstations 410 may function as an initiator workstation and another of the workstations 420 may function as a participant workstation. The initiator workstation 410 may initiate a request to collaborate with the participant workstation 420. For example, the collaboration request may be initiated automatically by the initiator workstation 410 or manually by an initiator (a user at the initiator workstation 410).

In an embodiment, the collaboration request from the initiator workstation 410 may be rejected or accepted by the participant workstation 420. For example, the collaboration request may be rejected or accepted automatically by the participant workstation 420 or manually by a participant (a user at the participant workstation 420). If the collaboration request is rejected, the participant workstation 420 may communicate a reject response to the initiator workstation 410, and the collaboration session may then be ended. If the collaboration request is accepted, the participant workstation 420 may communicate an accept response to the initiator workstation 410, and the collaboration may then be started.

In an embodiment, the initiator workstation 410 may select data, such as studies, reports, images, annotations, regions of interest, audio, video, text, and/or other information, to be shared with the participant workstation 420. For example, the initiator workstation 410 may share data automatically based at least in part on one or more rules and/or preferences, or manually based at least in part on input from the initiator.

In an embodiment, the initiator workstation 410 may manipulate the shared data. For example, the initiator workstation 410 may manipulate the shared data automatically based at least in part on one or more rules and/or preferences, or manually based at least in part on input from the initiator. Similarly, the participant workstation 420 may manipulate the shared data. For example, the participant workstation 420 may manipulate the shared data automatically based at least in part on one or more rules and/or preferences, or manually based at least in part on input from the participant.

In an embodiment, the initiator workstation 410 and the participant workstation 420 may manipulate the shared data simultaneously or substantially simultaneously. That is, the initiator workstation 410 and the participant workstation 420 may manipulate the shared data at the same time or within some delayed period of time based at least in part on system delay, processing delay, communication lag, and/or time needed by a user (the initiator and/or participant) to confirm the manipulation, for example. Additionally, the terms simultaneous(ly), substantially simultaneous(ly), contemporaneous(ly), substantially contemporaneous(ly), in real-time, and substantially in real-time may be used interchangeably to refer to the aforementioned manipulation of shared data.

In an embodiment, the initiator workstation 410 and the participant workstation 420 may display the shared data, including any manipulations thereof. More particularly, the initiator workstation 410 and the participant workstation 420 may display the same content and/or activity. For example, in a surgical planning session, a surgeon and a radiologist may view and annotate the same 2-D or 3-D image while discussing the proper placement of a stent.

In an embodiment, the shared data may be displayed simultaneously or substantially simultaneously. That is, the initiator workstation 410 and the participant workstation 420 may display the shared data at the same time or within some delayed period of time based at least in part on system delay, processing delay, communication lag, and/or time needed by a user (the initiator and/or participant) to confirm the display, for example. Additionally, the terms simultaneous(ly), substantially simultaneous(ly), contemporaneous(ly), substantially contemporaneous(ly), in real-time, and substantially in real-time may be used interchangeably to refer to the aforementioned manipulation of shared data.

In an embodiment, the initiator workstation 410 and the participant workstation 420 may manipulate the shared data in such a way as to cause conflicts (i.e., a race condition). For example, while viewing the 2-D or 3-D image, the surgeon and the radiologist from the previous example may attempt to place different annotations on the image at the same time and in the same location.

In an embodiment, the image and information management system 400 may resolve such conflicts based at least in part on one or more rules and/or preferences. For example, the image and information management system 400 may suspend all action until the conflict is resolved by the users (the initiator and/or participant). Additionally, for example, the image and information management system 400 may notify the users with an error message, and then suspend all action until the conflict is resolved, as described above. Alternatively, for example, the image and information management system 400 may resolve the conflict based on the priority of the action (first in time, last in time, initiator workstation 410, participant workstation 420, etc.). As the first participant to interact with contextual data initiates, others, depending on rules and privileges, may not interact concurrently. These users must then alert the system and initiator of their desire to interact. The initiator may then relinquish his/her interaction priority to that individual or others in the group. If the collaboration meeting relates to bitmap or graphical information, concurrent interaction may be possible. This would allow multiple users to simultaneously interact with "personalized" cursors to point out or annotate subtle anatomical information. In an embodiment, connection and collaboration between the initiator workstation 410 and the participant workstation 420 may occur regardless of display resolution (low resolution display, high resolution display, etc.) at the workstations 410, 420. For example, diagnostic images may be displayed at the initiator workstation 410 and/or the participant workstation 420 without regard to display resolution. Software and/or hardware running on the initiator workstation 410 and/or the participant workstation 420 may accommodate for differences in display resolution and may help to ensure that a diagnostic quality image is displayed. Furthermore, in an embodiment, connection and collaboration between the initiator workstation 410 and the participant workstation 420 may occur independent of the number of displays connected to each workstation 410, 420. For example, the image and information management system 400 may resolve display of information between an initiator workstation 410 with one or more displays and a participant workstation 420 with one or more displays.

In an embodiment, the initiator workstation 410 and the participant workstation 420 may include interfaces 412 and 422, respectively, for displaying and/or manipulating the shared data. The interfaces 412, 422 may include a graphical user interface (GUI), such as the graphical user interface 600 of Figure 6, a command line interface, and/or other interface, for example. The interfaces 412, 422 may be connected to an input device, such as a keyboard, mouse, touchpad, and/or other input device, for example.

In an embodiment, the initiator workstation 410 and the participant workstation 420 may include communication devices 414 and 424, respectively, for communication between the initiator workstation 410 and the participant workstation 420. The communication devices 414, 424 may include a modem, wireless modem, cable modem, Bluetooth^{™} wireless device, infrared communication device, wired communication device, and/or other communication device, for example. The communication devices 414, 424 communicate and transfer data via one or more communication protocols, such as the Digital Imaging and Communications in Medicine (DICOM) protocol. The communication devices 414, 424 coordinate with processors in the workstations 410, 420 to establish a connection between the workstations 410, 420 to share and/or manipulate data, for example. In an embodiment, the initiator workstation 410 and the participant workstation 420 may share and/or manipulate data over a network, such as a client-server, peer-to-peer, wireless, internet, and/or other type of network, for example.

In an embodiment, the initiator workstation 410 may interface with the participant workstation 420 according to one or more rules and/or preferences. For example, the rules and/or preferences may be based at least in part on contextual patient information.

In an embodiment, a password and/or other authentication, such as voice or other biometric authentication, may be used to establish a connection between the initiator workstation 410 and the participant workstation 420.

In an embodiment, the image and information management system 400 may include additional security features. For example, the image and information management system 400 may include data encryption and/or digital certificates. Additionally, for example, the image and information system 400 may include logging and tracking features for compliance with patient privacy standards, such as the Health Insurance Portability and Accountability Act (HIPAA).

In an embodiment, users at the workstations 410, 420 may communicate via telephone, electronic "chat" or messaging, Voice over Internet Protocol (VoIP) communication, and/or other communication via the workstations 410, 420 and/or separate from the workstations 410, 420. Users at the workstations 410, 420 may share display protocols, perspectives, rules, information, etc.

In an embodiment, the initiator workstation 410 and/or the initiator may save the shared data, including any manipulations thereof. Furthermore, in an embodiment, the initiator workstation 410 and/or the initiator may allow the participant workstation 420 and/or the participant to save any or all of the shared data, including any manipulations thereof.

In an embodiment, the initiator workstation 410 and/or the initiator may end the collaboration session with any or all of the participant workstations 420.

Additionally, the participant workstation 420 and/or the participant may end the collaboration session, but only with the initiator workstation 410. The participant workstation 420 and/or the participant may not end the collaboration session with other participant workstations 420.

In an embodiment, one or more initiator workstations 410 may communicate with one or more participant workstations 420. The initiator workstation 410 or other components of the image and information management system 400 may store profile(s) and/or other connection information for one or more participant workstations 420 or participants. In an embodiment, interaction between the initiator workstation 410 and the participant workstation 420 is manually initiated. In an embodiment, interaction between the initiator workstation 410 and the participant workstation 420 may be scheduled based on calendar or availability information, user preference, rules, and/or other criteria, for example. In an embodiment, the participant workstation 420 is automatically detected by the initiator workstation 410. In an embodiment, a certain type of initiator workstation 410, such as a PACS workstation, may communicate with and control a different type of participant workstation 420, such as a HIS, RIS, CIS, CVIS, LIS, or EMR workstation.

In an embodiment, a healthcare practitioner may use the initiator workstation 410 to collaborate with another healthcare practitioner at the participant workstation 420. For example, a radiologist at an initiator workstation 410 may indicate findings within image data to a physician at a participant workstation 420. A healthcare practitioner may also convey and/or identify diagnosis information, treatment information, and/or consultation or referral information, for example. For example, a surgeon may consult a specialist in real-time or substantially real-time during surgery and allow the specialist to view and comment on images and/or data from the operation in progress. In an embodiment, a healthcare practitioner at the initiator workstation 410 and/or the participant workstation 420 may dictate a report and/or annotate an image. In an embodiment, functions at the initiator workstation 410 and/or the participant workstation 420 may be controlled via voice command.

In an embodiment, the participant workstation 420 may share data with the initiator workstation 410. For example, a radiologist at a participant workstation 420 may share a 2-D or 3-D image with a physician at an initiator workstation 410.

In an embodiment, the participant workstation 420 and the initiator workstation 410 may manipulate the newly shared data simultaneously or substantially simultaneously. For example, the radiologist and the physician of the previous example may annotate the image at the same time.

In an embodiment, the participant workstation 420 and the initiator workstation 410 may display the newly shared data, including any manipulations thereof. For example, the radiologist and the physician of the previous example may display the image and annotations thereof.

In an embodiment, the participant workstation 420 serves or functions as an initiator workstation and the initiator workstation 410 serves or functions as a participant workstation with respect to the newly shared data.

In an embodiment, any workstation in the image and information management system 400 may serve or function as an initiator workstation 410 and/or a participant workstation 420 with respect to any other workstation.

In an embodiment, the initiator workstation 410 and the participant workstation 420 may include any type of computer and/or processor, and are not limited to workstations. For example, the workstations 410, 420 may include personal computers.

In an embodiment, the image and information management system 400 may include any type of remote conference and/or collaboration system, and is not limited to an image and information management system. For example, the system 400 may include two personal computers connected over the internet.

The image and information management system 400 may be implemented in software, hardware, and/or firmware.

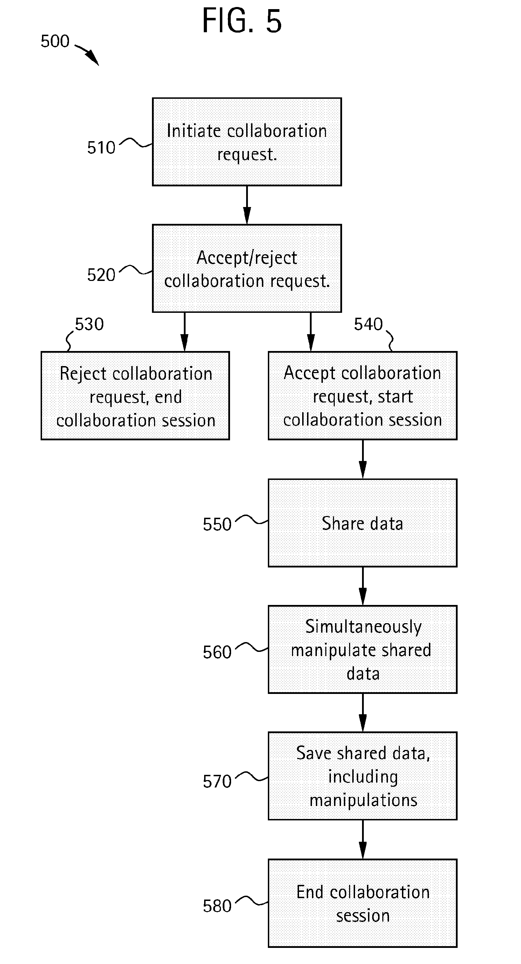

Figure 5 illustrates a flow diagram of a method 500 for simultaneous collaboration between workstations in accordance with an embodiment of the present invention.

At step 510, an initiator workstation, such as the initiator workstation 410 of Figure 4, may initiate a request to collaborate with a participant workstation, such as the participant workstation 420 of Figure 4. The collaboration request may be initiated, for example, automatically by the initiator workstation or manually by an initiator (a user at the initiator workstation). For example, a surgeon at a PACS workstation may initiate a request to collaborate with a radiologist at another PACS workstation.

At step 520, the participant workstation may reject or accept the collaboration request. The collaboration request may be rejected or accepted, for example, automatically by the participant workstation or manually by a user at the participant workstation. For example, a surgeon at the PACS workstation may reject or accept a collaboration request from a radiologist at another PACS workstation.

At step 530, the collaboration request may be rejected. If the collaboration request is rejected, the participant workstation may communicate a reject response to the initiator workstation, and the collaboration session may then be ended. In an embodiment, the collaboration request may be rescheduled and/or another participant workstation may be contacted.

At step 540, the collaboration request may be accepted. If the collaboration request is accepted, the participant workstation may communicate an accept response to the initiator workstation, and the collaboration session may then start.

At step 550, the initiator workstation may select data, such as studies, reports, images, annotations, regions of interest, audio, video, text, and/or other information, to share with the participant workstation. Additionally, the initiator workstation and the participant workstation may display the shared data, including any manipulations thereof, as described below in step 560.

At step 560, the initiator workstation and the participant workstation may manipulate the shared data simultaneously or substantially simultaneously. For example, in a surgical planning session, a surgeon and radiologist may view and annotate the same 2-D or 3-D image while communicating about proper placement of a stent.

At step 570, the initiator workstation may save the shared data, including any manipulations thereof. Furthermore, in an embodiment, the initiator workstation may allow the participant workstation to save any or all of the shared data, including any manipulations thereof.

At step 580, the initiator workstation may end the collaboration session. For example, an initiator workstation may communicate an end request to the participant workstation, and the collaboration session may then be ended. In an embodiment, the participant workstation may end the collaboration session, but only with the initiator workstation. The participant workstation may not end the collaboration session between the initiator workstation and other participant workstations.

As will be appreciated by those of skill in the art, certain steps may be performed in ways other than those recited above and the steps may be performed in sequences other than those recited above.

Additionally, the steps 510-580 of the method 500 of Figure 5 may be introduced into the image and information system 400 of Figure 4, the Picture Archiving and Communication System (PACS) 100 of Figure 1, and/or other remote conference and/or collaboration system (e.g., two personal computers connected over the internet) as a set of instructions on a computer-readable storage medium, such as a floppy disk or a hard drive, for example. The set of instructions may be implemented using software, hardware, and/or firmware, for example.

Figure 6 illustrates a graphical user interface 600 for an image and information management system with remote conferencing and collaboration capability in accordance with an embodiment of the present invention.

In an embodiment, the graphical user interface 600 may be the graphical user interfaces 212, 222 of Figure 2 and/or the graphical user interfaces 412, 422 of Figure 4, as described above.

The graphical user interface 600 includes a participant window 610, a audio/video (A/V) conference widow 620, and a shared data display window 630. The graphical user interface 600 may also include a text conference window 640 (not shown).

In an embodiment, the participant window 610 of the graphical user interface 600 may include a list of participants, such as surgeons, radiologists, anesthesiologists, medical internists, clinicians, physicians, and/or patients.

In an embodiment, the participant window 610 may include information about the participants, such as information regarding identification, availability, connectivity, and/or other relevant participant information.

In an embodiment, the participant window 610 may identify one or more potential participants in a remote conference or collaboration session. For example, a participant may be identified by name, occupation, facility, location, and/or other relevant participant information. Additionally, for example, a participant may be identified as Joan Stem, MD, Surgeon, Pleasant Valley Hospital, Mark Addonis, MD, Anesthesiologist, Boston Anesthesia Associates, or Yuko Nogi, MD, Medical Internist, Hope County Internists, LLC.

In an embodiment, the participant window 610 may indicate a participant's availability for a remote conference or collaboration session. For example, a participant may select a pre-defined availability status, such as online, offline, busy, be right back, out to lunch, and/or other relevant message. Alternatively, for example, a participant may create a custom availability message.

In an embodiment, the participant window 610 may indicate a participant's connectivity status. For example, a participant that is connected to a remote conference or collaboration session may be presented in bold and/or placed near the front and/or top of the participant window 610. Conversely, a participant that is not connected to a remote conference or collaboration session may be presented in shadow and/or near the back and/or bottom of the participant window 610. Additionally, for example, webcam, instant messenger, and telephone icons may indicate that a participant is connected to a remote conference or collaboration session by webcam, instant messenger, and telephone, respectively.

In an embodiment, an initiator may add and/or remove participants to/from the participant window 610. In an embodiment, the participant window 610 may include a buddy list, such as the AOL™ buddy list and/or the MSN™ buddy list. In an embodiment, the graphical user interface 600 may include one or more participant windows 610. The participant windows 610 may be arranged based on identification, availability, connectivity, and/or other relevant information about the participant(s), as described above.

In an embodiment, the audio/video conference window 620 may include an audio feed and/or a video feed from one or more participants in a remote conference or collaboration session. The audio feed and/or video feed may be generated by a webcam, microphone, telephone, and/or other audio/video device. In an embodiment, two or more participants may communicate with different audio/video devices. For example, a surgeon with a webcam may be able to communicate both an audio feed and a video feed, but an anesthesiologist with a telephone may only be able to communicate an audio feed.

In an embodiment, an initiator may communicate with a participant through the audio/video conference window 620. Additionally, one or more participants may communicate with the initiator and/or other participants through the audio/video conference window 620.

The graphical user interface 600 may also include a text conference window 640. The text conference window 640 is similar to the audio/video conference window 620, except that text may be the medium, as opposed to audio and/or video. The text conference window 640 may include a chat or instant message window, such as the AOL™ instant messenger and/or the MSN™ instant messenger, for example.

In an embodiment, the shared data display area 630 of the graphical user interface 600 may include shared data, such as studies, reports, images, annotations, regions of interest, audio, video, text, and/or other information.

In an embodiment, an initiator may manually select data to be displayed and shared with one or more participants in a remote conference or collaboration session. More particularly, the shared data may manually share and display data in the shared data display area 630. Furthermore, the initiator may arrange and/or aggregate the shared data under one or more pre-defined and/or user-defined tabs, for example.

In an embodiment, an initiator and/or an initiator workstation, such as the initiator workstation 210 of Figure 2 and/or the initiator workstation 410 of Figure 4, may automatically select data to be displayed and shared with one or more participants in a remote conference or collaboration session. More particularly, the initiator workstation may automatically display and share data in the shared data display area 630 based at least in part on one or more rules and/or preferences. The rules and/or preferences may be based at least in part on contextual patient information (e.g., symptoms, diagnoses, and/or participants in a remote conference or collaboration session). For example, if a surgeon, radiologist, and anesthesiologist are participating in a remote conference or collaboration session, key images, drug metabolism rates, and allergies may be automatically displayed and shared based at least in part on one or more rules and/or preferences identifying these particular participants in the context of a surgical planning session.

In an embodiment, a participant may select data to be displayed and shared with an initiator and the other participants in a remote conference or collaboration session. More particularly, a participant may display and share data by dragging and dropping the data into the shared data display area 630.

In an embodiment, an initiator may control the particular data that is shared with a particular participant. For example, in a surgical planning session between a surgeon, radiologist, and anesthesiologist, the surgeon (initiator) may display and share 2-D and 3-D images only with the radiologist, and drug metabolism rates and allergies only with the anesthesiologist.

The graphical user interface 600 may be implemented in software, hardware, and/or firmware, for example.

The initiator may also be a participant, and thus has all rights and privileges of a participant, as well as the additional rights and/or privileges of an initiator.

Certain embodiments allow "smart" collaboration for dynamic sharing of contextual patient information. Certain embodiments allow users control in sharing information to specific participants. Certain embodiments allow users to view and interact with relevant information rather than presenting the entire system or navigating to specific information kernels. Certain embodiments allow users immediate access to contextual patient information without searching or navigating the entire system.

Certain embodiments allow users to share specific information within a chat session as opposes to an entire system. Certain embodiments allow users to view shared patient context without having to log-in to disparate systems simultaneously. Certain embodiments allow users to interactively share information by dragging and dropping into the tabular field. Certain embodiments allow users to discuss contextual patient information in real-time without simultaneous navigation.

While the invention has been described with reference to certain embodiments, it will be understood by those skilled in the art that various changes may be made and equivalents may be substituted without departing from the scope of the invention. In addition, many modifications may be made to adapt a particular situation or material to the teachings of the invention without departing from its scope. Therefore, it is intended that the invention not be limited to the particular embodiment disclosed, but that the invention will include all embodiments falling within the scope of the appended claims.

**Parts List**

| | |
|---|---|
| PAC S System | 100 |
| Imaging Modality | 110 |
| Acquisition Workstation | 120 |
| Network Server | 130 |
| Display Workstation | 140 |
| Image and Information Management System | 200 |
| Workstation | 210,220 |
| Interface | 212, 222 |
| Communication Device | 214, 224 |
| Flow Diagram | Figure 3 |
| Image and Information Management System | 400 |
| Workstation | 410,420 |
| Interface | 412, 422 |
| Communication Device | 414, 424 |
| Flow Diagram | Figure 5 |
| Graphical User Interface | 600 |
| Participant Window | 610 |
| Audio/Video Communication Window | 620 |
| Shared Data Display Window | 630 |
| Text Communication Window | 640 |

## Claims

1. A system (400) for improved conferencing and collaboration in a healthcare environment, the system (400) including:
an initiator workstation (410), wherein the initiator workstation (410) is capable of sharing data over a network with a participant workstation (420) and manipulating the shared data based at least in part on input from an initiator; and
the participant workstation (420), wherein the participant workstation (420) is capable of manipulating the shared data based at least in part on input from a participant,
wherein the initiator workstation (410) and the participant workstation (420) are capable of simultaneous user manipulation of the shared data.

2. The system (400) of claim 1, wherein the initiator and participant workstations (410, 420) include first and second picture archiving and communications system workstations.

3. The system (400) of claim 1 or claim 2, wherein the initiator and participant workstations (410, 420) include first and second personal computers.

4. The system (400) of any preceding claim, wherein the shared data includes at least one of a medical study, a report, a diagnostic image, an image annotation, a region of interest in an image, audio, and video.

5. The system (400) of any preceding claim, wherein the shared data is manipulated by an input device.

6. The system (400) of any preceding claim, wherein the shared data is manipulated by voice command.

7. The system (400) of any preceding claim, wherein the network includes at least one of a client-server network, a peer-to-peer network, a wireless network, and the Internet.

8. The system (400) of any preceding claim, wherein the initiator and participant workstations (410, 420) are capable of displaying the shared data.

9. A method (500) for improved conferencing and collaboration in a healthcare environment, the method including:
initiating a collaboration session, wherein the collaboration session includes sharing data over a network between an initiator workstation (410) and a participant workstation (420); and
allowing manipulation of the shared data simultaneously in the collaboration session based at least in part on input from an initiator and a participant.

10. The method (500) of claim 9, further including displaying the simultaneous user manipulations.
